# FASCICULE DE BREVET EUROPEEN

(11) **EP 2 601 167 B1**
(45) Date de publication et mention de la délivrance du brevet: **12.09.2018**
(21) Numéro de dépôt: 11736026.3
(22) Date de dépôt: 06.07.2011
(51) Int. Cl.: C07C 49/813, C07C 49/83, C07C 309/44, C08G 65/40, H01M 8/1025, C08L 71/00, H01M 8/1039, H01M 8/1088

(54) **MONOMÈRE PERFLUOROALCANE AROMATIQUE**
AROMATISCHES PERFLUORALKANMONOMER
AROMATIC PERFLUOROALKANE MONOMER

(30) Priorité: 04.08.2010 FR 1056440
(43) Date de publication de la demande: 12.06.2013
(73) Titulaire: Compagnie Générale des Etablissements Michelin, 63000 Clermont-Ferrand (FR)
(72) Inventeur: FEDURCO, Milan, F-63040 Clermont-ferrand Cedex 9 (FR)
(74) Mandataire: Desbordes, Guillaume
(86) Numéro de dépôt international: PCT/EP2011/061429
(87) Numéro de publication internationale: WO 2012/016780

(56) Documents cités:
- US-A1- 2005 221 135
- US-B1- 6 495 209
- US-B1- 7 037 614

## Description

### I. DOMAINE DE L'INVENTION

La présente invention est relative aux monomères utilisables pour la synthèse de polymères destinés notamment, sous forme sulfonée, à constituer un électrolyte solide ou membrane dans une pile à combustible.

Elle est plus particulièrement relative aux monomères ci-dessus du type aromatiques et comportant une unité structurelle centrale du type perfluoroalkylène.

### II. ETAT DE LA TECHNIQUE

L'intérêt récent pour les piles à combustible vient de leur capacité à convertir l'énergie chimique en électricité avec un rendement relativement élevé et une émission basse de polluants environnementaux. Aujourd'hui, l'utilisation de tels dispositifs électrochimiques s'étend de l'industrie automobile aux ordinateurs portatifs, aux téléphones cellulaires, à la génération stationnaire d'énergie électrique, ainsi qu'à d'autres applications comprenant l'exploration de la mer et de l'espace.

On rappellera tout d'abord qu'une pile à combustible est un générateur d'énergie électrochimique dans lequel est entretenue sous contrôle une réaction chimique entre l'hydrogène et l'oxygène qui va produire de l'eau (réaction inverse de l'électrolyse). Elle produit de l'énergie électrique et de la chaleur. L'électrolyte y est constitué typiquement d'une membrane polymère PEM (abréviation pour *"Polymer Electrolyte Membrane*") conductrice de protons et apte à séparer les espèces réactives, constituée de deux nanophases bien distinctes : d'une part une partie hydrophobe assurant l'intégrité mécanique, étanche à l'eau et aux gaz (H₂ et O₂), d'autre part une partie sulfonée constituée de canaux hydrophiles étroits permettant le passage des protons et assurant donc la conductivité ionique de la pile. Cette membrane polymère est disposée entre l'anode et la cathode de la pile, un tel assemblage étant communément appelé "MEA" (pour *Membrane Electrode Assembly*).

De tels piles à combustible, assemblages MEA ainsi que leurs principes généraux de fonctionnement sont bien connus, ils ont été décrits dans un très grand nombre de documents ; à titre d'exemples, on peut citer l'article général intitulé *"*Functional fluoropolymers for fuel cell membranes" de Renaud Souzy & Bruno Ameduri, Prog. Polymer Sci. 30 (2005), 644-687, ainsi que les demandes de brevet WO 2005/006472, WO 2006/012953, WO 2006/012954, WO 2006/100029, WO 2008/125174.

Un matériau polymérique bon candidat pour une pile à combustible PEM doit satisfaire à de très hautes exigences en ce qui concerne ses propriétés mécaniques, physiques et chimiques. Idéalement, on attend de l'assemblage MEA qu'il puisse fonctionner pendant des milliers d'heures à des températures relativement élevées (60 à 100°C dans le cas de piles PEM, jusqu'à 160°C dans le cas de piles au méthanol dites DMFC) tout en étant exposé à une humidité particulièrement élevée et des valeurs de pH acide proches de zéro. La plupart des polymères connus subissent une décomposition sous de telles conditions, qu'ils soient de type aliphatiques comme aromatiques.

Des copolymères aliphatiques dérivés d'acide perfluorosulfonique, commercialisés par exemple sous le nom de Nafion® ou Flemion®, ont été employés intensivement comme membranes conductrices dans des piles à combustible du type hydrogène/ air, hydrogène/ oxygène ou méthanol/ air.

Malgré une très bonne conductivité ionique et une haute stabilité chimique, l'emploi de polymères du type Nafion® n'est tout d'abord pas adapté à une utilisation dans des piles à combustible du type méthanol, cela en raison d'une performance réduite pour les températures d'utilisation les plus élevées, due à une augmentation importante de perméabilité de la membrane vis-à-vis du méthanol.

Un autre inconvénient connu des polymères du type Nafion®, en fonctionnement dans la pile, est leur stabilité chimique relativement limitée. En effet, les polymères perfluorés sont connus pour absorber des quantités importantes d'eau responsables de gonflements et de changements dimensionnels répétés de la membrane : des cycles répétés de séchage et d'humidification, lors des arrêts et démarrages successifs de la pile à combustible, entraînent une perméabilité accrue aux gaz (H₂ et O₂) ; cette perméabilité accrue est responsable de la formation d'eau oxygénée et de radicaux libres (OH), autant de mécanismes qui peuvent conduire à une dégradation rapide de la membrane et à une fin de vie prématurée de la pile à combustible. Pour limiter ces changements dimensionnels et améliorer ainsi l'endurance des membranes, il a notamment été proposé d'ajouter, à titre de polymère de renforcement, un second polymère fluoré, notamment un PTFE (polytétrafluoroéthylène) du type microporeux expansé (ou "ePTFE"), et de constituer ainsi des membranes composites plus endurantes (voir par exemple US 6 495 209).

Enfin, un autre inconvénient majeur des polymères du type Nafion® est leur coût de synthèse, sans parler d'une chimie de base qui ne répond plus aujourd'hui aux exigences les plus récentes en termes d'environnement, de règles d'hygiène et sécurité.

Aussi, de nombreuses recherches ont été conduites dans le passé pour tenter de réduire le coût des membranes PEM.

Il a été proposé notamment de remplacer les polymères aliphatiques ci-dessus par des polymères aromatiques, de coût inférieur et qui ont par ailleurs comme avantage de présenter une perméabilité aux gaz (H₂ et O₂) qui est réduite.

Des exemples de tels polymères sont par exemple des poly(arylène-éther-sulfone), commercialisés notamment sous les dénominations "Udel", "Radel" ou encore des poly (éther-éther-cétone) commercialisés par exemple sous dénomination "PEEK". Les polymères aromatiques ci-dessus, une fois sulfonés, ne permettent pas encore aujourd'hui d'atteindre le compromis de performances et de coût offert avec les polymères aliphatiques fluorés du type Nafion®. En outre, ces polymères aromatiques se mélangent généralement mal avec un polymère type ePTFE et les membranes qui en sont issues ne peuvent donc être renforcées facilement par un polymère ePTFE, un tel renforcement exigeant un traitement de surface préalable du polymère ePTFE par plasma ou par voie chimique dans des milieux chimiques très agressifs (voir par exemple article intitulé *"*Challenging reinforced composite polymer electrolyte membranes based on disulfonated poly(arylene-ether-sulfone)-impregnated expanded PTFE for fuel cell applications", Xiaobing Zhu et al, J. Mat. Chem., 2007, 386-397).

D'autres exemples de polymères du type aromatiques ont été décrits plus récemment dans les documents brevet US2005/0221135 et US 7037614. Il s'agit de polymères triazine sulfonés dont les monomères sont connectés par des ponts éther (-O-). Les synthèses décrites dans ces documents sont complexes, coûteuses et difficiles à reproduire. On a constaté en outre que leur stabilité, chimique et dimensionnelle, est insuffisante même après un traitement final de réticulation des membranes, traitement qui nécessite par ailleurs une autre chimie complexe et coûteuse.

### III. BREVE DESCRIPTION DE L'INVENTION

Au cours de leurs recherches, les Demanderesses ont trouvé un monomère aromatique nouveau, plus précisément un monomère perfluoroalcane aromatique spécifique, qui est utilisable pour la synthèse d'une membrane polymère permettant de pallier, au moins en partie, les inconvénients précités.

Ce monomère perfluoroalcane aromatique de l'invention est tel qu'énoncé en revendication 1.

A partir de ce monomère conforme à l'invention, il s'est avéré possible de synthétiser un polymère qui, comparé aux polymères de l'art antérieur précédemment décrits, possède une stabilité chimique et une résistance à l'oxydation nettement améliorées. Il permet de fabriquer des membranes PEM qui de manière inattendue, comparées à des membranes commerciales du type Nafion® développées depuis déjà longtemps, présentent une stabilité chimique et dimensionnelle au moins équivalentes, une conductivité ionique approchant celle de ces membranes commerciales. Enfin, ce qui n'est pas son moindre avantage, le polymère issu du monomère de l'invention peut être rendu compatible avec un polymère microporeux ePTFE pour un renforcement optimal de la membrane, sans nécessiter les traitements de surface qui ont été évoqués supra.

L'invention concerne également un procédé de synthèse d'un polymère par polycondensation d'au moins un monomère perfluoroalcane aromatique conforme à l'invention.

L'invention concerne également l'utilisation d'un monomère perfluoroalcane aromatique conforme à l'invention pour la fabrication d'une membrane de polymère utilisable dans une pile à combustible du type PEM.

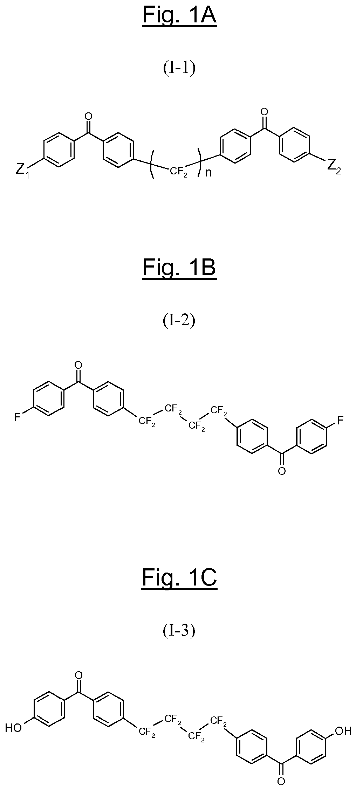

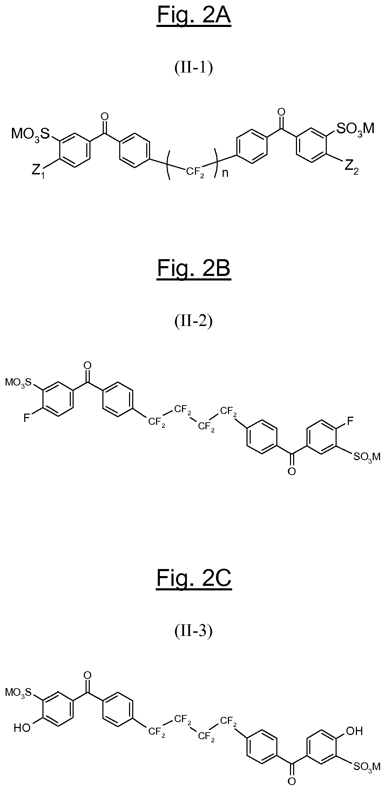

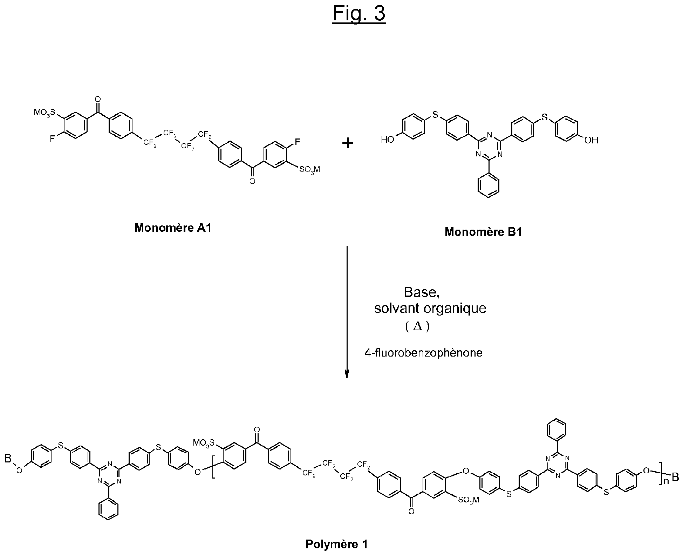

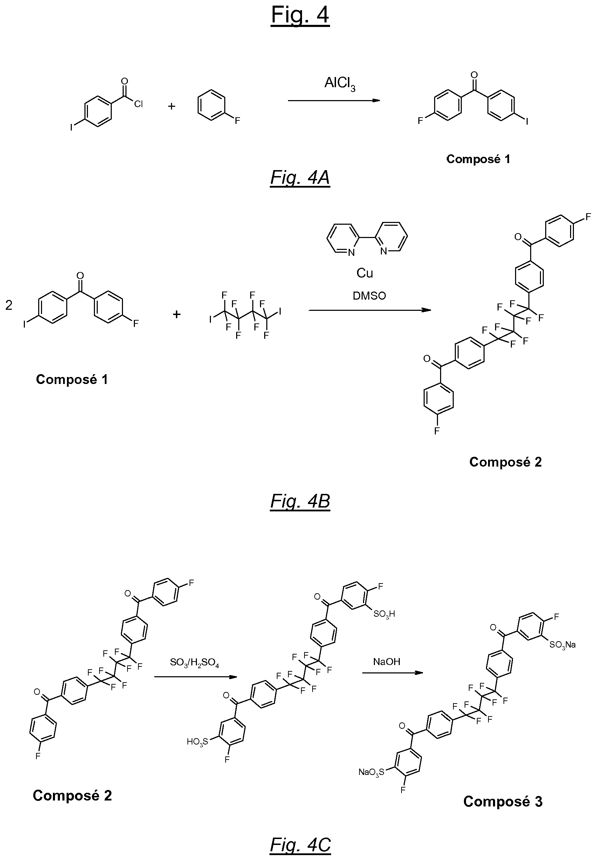

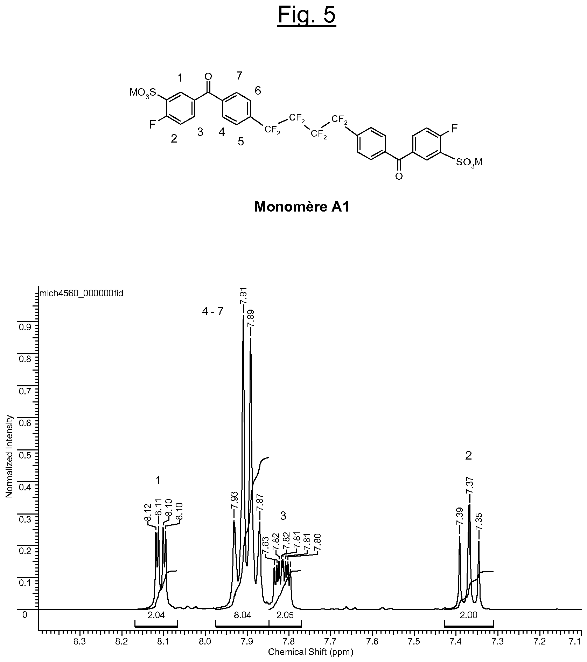

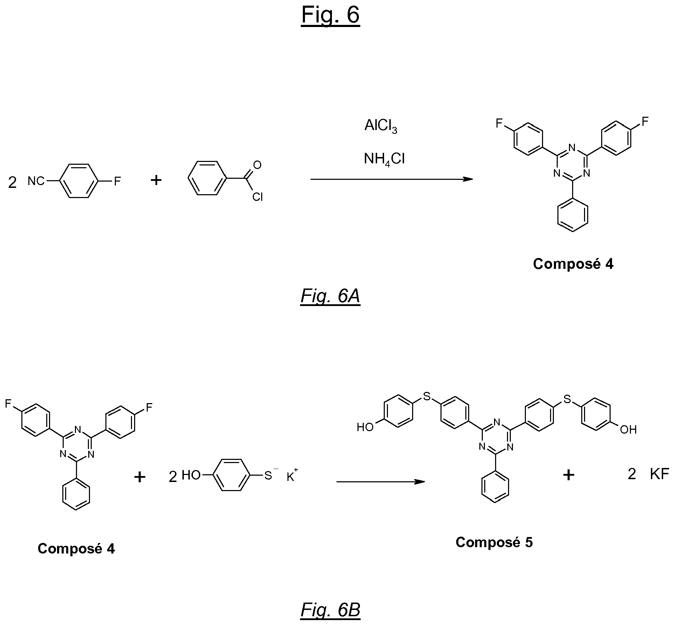

L'invention ainsi que ses avantages seront aisément compris à la lumière de la description détaillée et des exemples de réalisation qui suivent, ainsi que des figures relatives à ces exemples qui représentent ou schématisent :
- des exemples de monomères conformes à l'invention de formule (I), de formules particulières respectives (I-1), (I-2) et (I-3) (Fig. 1A, 1B et 1C) ;
- des exemples de monomères conformes à l'invention de formule (I), de formules particulières respectives (II-1), (II-2) et (II-3) (Fig. 2A, 2B et 2C) ;
- un exemple de polymère (Polymère 1) ainsi qu'un schéma de synthèse possible de ce polymère par polycondensation d'un monomère A1 conforme à l'invention avec un second monomère B1 non conforme à l'invention (Fig. 3) ;
- un schéma de synthèse possible, en trois étapes successives, du monomère A1 (ou Composé 3) conforme à l'invention (Fig. 4) ;
- le spectre RMN ¹H (500 MHz) du monomère A1 (ou Composé 3) dissous dans DMSO-*d₆* (Fig. 5) ;
- un schéma de synthèse possible, en deux étapes successives, du monomère B1 (ou Composé 4) non conforme à l'invention (Fig. 6) ;
- les courbes de polarisation comparées d'une pile à combustible PEM utilisant la membrane issue du Polymère 1 (courbe C_{A}) et une membrane commerciale (courbe C_{B}) (Fig. 9).

### IV. DESCRIPTION DETAILLEE DE L'INVENTION

Le monomère perfluoroalcane aromatique de l'invention a donc pour caractéristique essentielle de répondre à la formule (I) de la revendication 1.

Autrement dit, le monomère perfluoroalcane de formule (I) ci-dessus est un monomère perfluoroalcane benzophénone ayant pour formule développée : 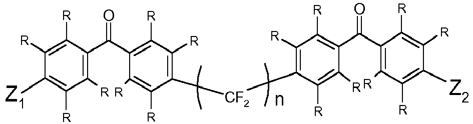 dans laquelle R représente l'hydrogène.

Dans la formule (I) ci-dessus et toutes les variantes préférentielles de l'invention décrites dans la présente demande, n varie de 2 à 8 ; De préférence, le monomère perfluoroalcane de l'invention est un monomère perfluorobutane c'est-à-dire que n est égal à 4.

Les groupes Z₁ et Z₂ correspondent au groupe hydroxyle ou à un halogène, en particulier fluor ou chlore.

Ainsi, selon un premier mode de réalisation particulièrement préférentiel, les groupes Z₁ et Z₂ correspondent à l'halogène fluor dans la formule (I). Dans le cas particulier plus préférentiel où le bloc central perfluoroalkylène est un perfluorobutylène, le monomère perfluoroalcane aromatique de l'invention est donc le 1,4-bis-(4-fluorobenzophénone)-perfluorobutane, répondant à la formule (I-2) représentée à la figure 1B annexée, dans laquelle les groupes phénylène peuvent être substitués ou non substitués.

Selon un deuxième mode de réalisation particulièrement préférentiel, les groupes Z₁ et Z₂ correspondent à l'hydroxyle dans la formule (I). Ainsi, dans le cas particulier plus préférentiel où le bloc central perfluoroalkylène est un perfluorobutylène, le monomère perfluoroalcane de l'invention aromatique de formule (I) est donc le 1,4-bis-(4-hydroxybenzophénone)-perfluorobutane répondant à la formule (I-3) représentée à la figure 1C annexée, dans lesquelles les groupes phénylène peuvent être substitués ou non substitués.

Le monomère perfluoroalcane aromatique conforme à l'invention précédemment décrit est avantageusement utilisable pour la synthèse de polymères pouvant constituer, sous forme sulfonés, un électrolyte (ou membrane, ce qui est équivalent) dans une pile à combustible. Par polymère doit être entendu tout homopolymère ou copolymère, notamment copolymère à blocs, comportant au moins des unités structurelles issues du monomère de l'invention.

On entend par "monomère sulfoné" ou "polymère sulfoné", par définition et de manière bien connue, un monomère ou polymère porteur d'un ou plusieurs groupes sulfoniques (-SO₃H), sulfonates (-SO₃M), ou mélanges de tels groupes, M représentant un cation de métal alcalin choisi préférentiellement parmi lithium (Li), cesium (Cs) sodium (Na) et potassium (K), plus préférentiellement parmi sodium (Na) et potassium (K). On rappellera brièvement ici que ce sont les groupes sulfoniques qui dans une pile PEM assurent la conductivité protonique du polymère utilisé comme membrane.

L'invention concerne particulièrement un monomère perfluoroalcane tel que précédemment décrit dans lequel au moins un groupe sulfonique ou sulfonate est porté par au moins l'un de ses groupes phénylène ou, le cas échéant, par au moins l'un des substituants de ses groupes phénylène. Par groupe phénylène porteur, il faut donc entendre dans la présente demande que le groupe phénylène lui-même ou l'un des éventuels substituants de ses atomes d'hydrogène est porteur d'un groupe sulfonique ou sulfonate.

Les figures 2A, 2B et 2C illustrent des exemples de monomères selon l'invention sous forme sulfonés dans lesquels au moins un groupe phénylène par groupe benzophénone est porteur d'un groupe sulfonate -SO₃M (M représentant un cation de métal alcalin, choisi préférentiellement parmi Li, Cs, Na et K, plus préférentiellement parmi Na et K) :
- un monomère perfluoroalcane aromatique sulfoné de formule (II-1) (Fig. 2A) ;
- un sel de métal alcalin de 1,4-bis-(4-fluorobenzophénone) perfluorobutane disulfonate, dans lequel les groupes Z₁ et Z₂ correspondent à l'halogène fluor et le bloc central perfluoroalcane est un perfluorobutylène (Fig. 2B) ;
- un sel de métal alcalin de 1,4-bis-(4-hydroxybenzophénone) perfluorobutane disulfonate, dans lequel les groupes Z₁ et Z₂ correspondent à l'hydroxyle et le bloc central perfluoroalkylène est un perfluorobutylène (Fig. 2C).

La figure 3 annexée représente un exemple de polymère synthétisable à partir d'un monomère perfluoroalcane aromatique conforme à l'invention, ainsi qu'un schéma de synthèse possible de ce polymère à partir d'un tel monomère.

Le polymère triazine (ci-après dénommé "Polymère 1") tel que représenté à la figure 3 (sous forme sulfoné) est constitué de deux types d'unités structurelles reliées entre elles par des ponts éther (-O-). Ce Polymère 1 peut être préparé par polycondensation d'un monomère conforme à l'invention noté A1 (ici, sous forme disulfoné) avec un monomère triazine non conforme à l'invention noté B1 à la figure 3, en présence d'une base et d'un solvant organique, selon un mode opératoire qui sera décrit en détail plus loin. Le monomère A1 conforme à l'invention correspond au monomère perfluorobutane aromatique de formule (II-2) précédemment décrit (Fig. 2B).

On note que ce Polymère 1 de la figure 3, sous forme sulfoné, est constitué d'unités structurelles à base perfluoroalcane aromatique (benzophénone) et d'unités triazine reliées entre elles par des ponts éther (-O-). Ici, le Polymère 1 comporte des extrémités de chaînes bloquées par des groupements bloquants benzophénone (notés B à la figure 3), hydrophobes et stériquement encombrants, destinés à réduire la solubilité du polymère dans l'eau.

### V. EXEMPLES DE REALISATION DE L'INVENTION

Les essais qui suivent décrivent tout d'abord en détail la synthèse des monomères A1 (conforme à l'invention) et B1 (non conforme à l'invention) puis celle du Polymère 1. Puis, le Polymère 1 est caractérisé et testé comme une membrane conductrice de protons dans une pile à combustible du type PEM.

Dans la présente description, sauf indication expresse différente, tous les pourcentages (%) indiqués sont des % en masse.

### V-1. Synthèse du monomère A1

Le monomère A1 est le 1,4-bis-(4-fluorobenzophénone)-perfluorobutane disulfoné, dont la formule est la suivante :

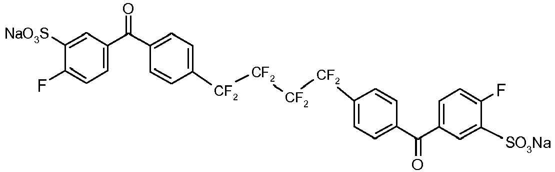

Ce monomère A1 conforme à l'invention (ou Composé 3) a été préparé selon le mode opératoire schématisé à la figure 4, en trois étapes successives, comme détaillé ci-après.

### V-1-A) Etape 1

Lors d'une première étape, on prépare le Composé 1 ou (4-fluoro-phényl)-(4-iodo-phényl)-méthanone, conformément à la figure 4A.

Dans un ballon préalablement séché de 250 ml sont ajoutés le 4-iodobenzoyl chlorure (30 g soit 112,6 mmol), le chlorure d'aluminium (15,0 g soit 112,7 mmol) et le fluorobenzène (21,7g soit 225, 8 mmol). Le mélange est agité à température ambiante sous un léger flux d'azote toute la nuit. Le lendemain, un solide est apparu et l'agitation n'est plus possible. On additionne alors 20 ml de fluorobenzène supplémentaires et les réactifs sont mélangés à 40°C (température à l'intérieur du ballon) pendant 3 h. L'appareillage est placé à 40°C sous vide (trompe à eau) et l'excès de fluorobenzène est distillé (pendant 30 min).

Directement dans le ballon de réaction sont ajoutés 200 g de glace, puis immédiatement 60 ml de HCl à 37%. Le produit solide ainsi obtenu est réduit en poudre dans un mortier en céramique, puis agité dans l'eau jusqu'à l'obtention d'une poudre blanche finalement séparée de la solution HCl par filtration (papier filtre) et lavée jusqu'à pH neutre. Le solide est séché à température ambiante (23°C) à l'aide de la trompe à eau, puis mélangé à 200 ml d'éthanol et finalement chauffé à 60°C (température à l'intérieur du ballon) jusqu'à ce que le tout soit dissous. Le composé est finalement précipité en refroidissant l'éthanol à température ambiante.

Le produit final (environ 30 g) est purifié par chromatographie sur silice (300 g) en utilisant un mélange hexane/acétate d'éthyle (rapport pondéral 16/4) comme phase mobile. Le produit est séparé de la phase mobile à l'évaporateur rotatif et séché à 80°C toute la nuit (sous vide). Le produit final de couleur crème (25 g) se révèle pur par analyse RMN et chromatographie TLC dans le mélange hexane/acétate d'éthyle (rapport 16/4), avec un point de fusion (mesuré par DSC) d'environ 137°C.

On obtient ainsi le Composé 1, de formule :

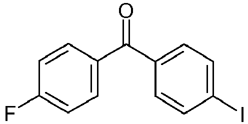

L'analyse RMN donne les résultats suivants :
*¹H NMR, 500 MHz (CD₂Cl₂): 7.17* - *7.20 (m, 2H), 7.48-7.50 (m, 2H), 7.80-7.82 (m, 2H), 7.87-7.89 (m, 2H).*

### V-1-B) Etape 2

Puis, au cours d'une seconde étape, on prépare le Composé 2 ou 1,4-bis-(4-fluorobenzophénone)-perfluorobutane, selon le mode opératoire qui suit et schématisé à la figure 4B.

Dans un ballon de 500 ml à 4 cols préalablement séché sont introduits 17,0 g de 4-iodo-4'-fluorobenzophénone (soit 52,13 mmol), 2,0 g de 2,2'-bipyridyl (soit 12,83 mmol) suivis de 11,83 g de 1,4-diiodoperfluorobutane (soit 26,06 mmol) et 150 ml de DMSO anhydre. Par la suite, 6,60 g de poudre de cuivre sont ajoutés et la solution est chauffée à 65°C (le bain d'huile est réglé à 74°C) pendant 5 h sous agitation constante et courant d'azote.

Le mélange réactionnel est refroidi à température ambiante puis versé dans 500 ml d'eau froide ; le produit précipite, puis il est filtré et dissous avec 1 litre de dichlorométhane. La phase organique est ensuite séchée avec Na₂SO₄ anhydre. Le produit final est purifié par chromatographie sur silice (300 g) dans un mélange dichlorométhane/cyclohexane (1/1).

On obtient ainsi le Composé 2 sous forme d'une poudre blanche, de formule :

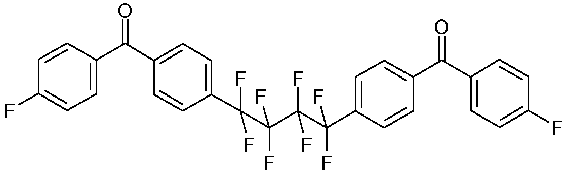

L'analyse RMN donne les résultats suivants :
*¹H NMR, 500 MHz (CD₂Cl₂): 7.19 - 7.23 (m, 2H), 7.73-7.75 (d, 4H), 7.83-7.87 (m, 8H).*
*¹⁹F NMR, 471.3 MHz (CDCl₃): 105.04 (m, 2F), 111.44-111.50 (d, 4F), 121.49-121.55 (m, 4F).*

La température de fusion du produit (mesurée par DSC) est égale à environ 222°C.

### V-1-C) Etape 3

Enfin au cours d'une troisième et dernière étape, on prépare le Composé 3 ou 1,4-bis-(4-fluorobenzophénone)-perfluorobutane disulfoné, selon le mode opératoire qui suit et schématisé à la figure 4C.

Le Composé 2 (2,5g soit 4,18 mmol) est placé dans un ballon de 50 ml à 4 cols préalablement séché l'aide d'un pistolet à air chaud et mis sous flux d'azote. 6 g d'acide sulfurique (distillé deux fois, Sigma-Aldrich) et 10 g d'oléum (65%, Merck) sont additionnés directement au solide. Le milieu réactionnel devient immédiatement foncé. La sortie des produits gazeux est purgée dans une trappe en verre vide suivie d'une trappe remplie de NaOH 30%. Le milieu réactionnel est chauffé à environ 130°C (environ 138°C dans le bain d'huile) pendant 4 h sous un flux moyen d'azote circulant au-dessus de la solution.

Une fois la sulfonation terminée, on laisse le mélange réactionnel se refroidir à température ambiante puis on le verse dans 63 g de glace et on laisse sous agitation. Une fois toute la glace fondue, 6,25g de NaCl sont additionnés. La solution est chauffée à 100°C puis refroidie à température ambiante afin que le monomère sulfoné précipite. Le précipité est ensuite dissous de nouveau dans 15 ml d'eau et chauffé à nouveau à 100°C pour le repasser sous forme liquide. Une fois que tout le produit s'est dissous, le pH est ajusté à 7,0 en additionnant goutte à goutte du NaOH (aq.) 10%. On laisse la solution se refroidir à température ambiante. Le solide blanc-crème ainsi obtenu est séparé de la phase aqueuse par filtration. Le produit est séché à 150°C toute la nuit (sous vide).

On obtient ainsi le Composé 3 (monomère A1), de formule :

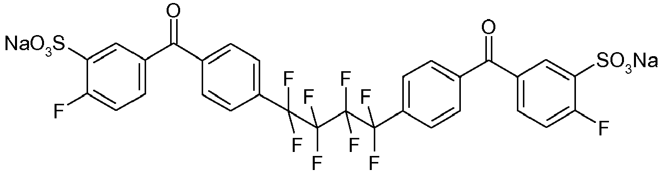

La figure 5 reproduit le spectre RMN ¹H (500 MHz) du monomère A1 ainsi obtenu, dissous dans DMSO-*d₆*.

L'analyse RMN donne les résultats suivants :
*¹H NMR, 500 MHz (DMSO-d₆): 7.35 - 7.39 (m, 2H), 7.80-7.83 (m, 2H), 7.87-7.937 (m, 8H), 8.09-8.10 (d, 1H), 8.11-8.12 (d, 1H).*

Enfin, la masse moléculaire du produit telle que mesurée par spectrométrie de masse "ESI" *(Electrospray Ionisation)* (mode négatif ; mélange eau/acétone 1/1) est égale à 778,9 (valeur théorique calculée égale à 779,6).

### V-2. Synthèse du monomère B1

Ce monomère B1 non conforme à l'invention (ou Composé 5 à la Fig. 6) a été préparé selon le mode opératoire schématisé à la figure 6, en deux étapes successives, comme détaillé ci-après.

### V-2-A) Etape 1

Lors d'une première étape, on prépare le Composé 4 ou 2,4-bis-(p-fluorophényl)-6-phényl-[1,3,5]-triazine, selon le mode opératoire qui suit et schématisé à la figure 6A.

Ce mode opératoire bien que différent s'inspire du procédé de synthèse de triphényl-triazines chlorées tel que décrit dans la publication de Spencer R.D. & Beggs B.H, "Determination of Four Closely Related Triaryl-s-Triazines by Infrared Spectroscopy", Anal. Chem. 1963, 31(11), 1633-1636.

Un ballon rond tricol de 500 ml équipé d'un barreau magnétique, d'un réfrigérant, d'un thermomètre, est séché à l'aide d'un pistolet à air chaud (l'appareillage est mis sous vide). 67,8 g de p-fluorobenzonitrile (0,56 mol) (Fluorochem 99%), 36,0 g de chlorure d'ammonium (0,68 mol), 34,0 g de chlorure d'aluminium (0,26 mol) et 32,0 g de chlorure de benzoyle (0,22 mol) sont placés dans le ballon sous azote. Le ballon est plongé dans un bain d'huile chauffé à 158°C et laissé toute la nuit à 150°C (température à l'intérieur du ballon de réaction), un léger flux d'azote placé au dessus du mélange réactionnel.

Le produit de réaction est refroidi à température ambiante (environ 23°C) et hydrolysé en additionnant 300 g de glace et 60 g de HCl 36%. Le solide est filtré, puis dispersé dans l'eau et lavé jusqu'à l'obtention d'un pH neutre. Le solide blanc est agité dans 500 ml de méthanol chauffé à reflux pendant 30 min, puis on laisse refroidir à température ambiante. Pour finir le produit est filtré et séché à 60°C sous vide.

On obtient ainsi 26,6 g (rendement 35%) de Composé 4, présentant un point de fusion (selon DSC) de 254,5°C.

L'analyse RMN donne les résultats suivants :
¹H NMR, 500 MHz (*CD₂Cl₂*): 7.30 - 7.34 (m, 4H), 7.62 - 7.65 (m, 2H), 7.68 - 7.70 (m, 1H), 8.79 - 8.80 (d, 2H), 8.82 - 8.85 (m, 4H).

### V-2-B) Etape 2

Lors d'une seconde étape, on prépare le Composé 5 ou 2,4-[(4-hydroxy-phénylsulfanyl phényl)]-6-(phényl)-[1,3,5]-triazine, selon le mode opératoire qui suit et schématisé à la figure 6B.

Le 4-hydroxythiophénol (ou 4-HTP) (99%, Acros) est conservé sous azote et sous forme solide. Le Composé 4 et K₂CO₃ sont séchés séparément toute la nuit à 150°C sous vide. Un barreau magnétique est placé dans un ballon rond de 21 (équipé d'un réfrigérant, d'un thermomètre et d'une entrée/sortie d'azote). L'appareillage est placé sous vide et séché. Une valve à deux voies est utilisée pour remplacer le vide par l'azote et purger continuellement avec le gaz inerte pendant l'addition des réactifs.

Le Composé 4 (9,13 g soit 26,44 mmol) et K₂CO₃ anhydre en poudre (9,69 g soit 1,2 eq. par rapport au 4-HTP) sont additionnés, pendant qu'il sont encore chauds (en sortie de séchage), dans l'appareillage purgé à l'azote. Ceci est suivi par l'addition de 750 ml de DMSO anhydre. La suspension obtenue est ensuite purgée pendant au moins 15 min avec un courant d'azote à l'intérieur de la solution.

La quantité requise de 4-HTP (7,45 g ou 58,42 mmol, soit 2,2 eq.), sous forme liquide, est transférée à l'aide d'une seringue en plastique de 10 ml, pesée directement à l'intérieur de la seringue et injectée dans le mélange réactionnel. Une fois que tous les réactifs sont additionnés, l'azote est purgé en continu au-dessus de la solution. Le mélange est chauffé à 100°C avec une agitation constante toute la nuit (20 heures), puis on laisse refroidir à température ambiante.

La purification du produit ne peut être faite en une seule étape : environ 250 ml de fraction aliquote de la réaction sont prélevés et versés dans une ampoule à extraction (3 litres) contenant 2,6 litres d'acétate d'éthyle/eau (rapport pondéral 1/1). Le reste du produit est gardé sous un flux constant d'azote. Le mélange placé dans l'ampoule à extraction est secoué (la couleur change du orange au jaune-citron) et le produit désiré est extrait dans la phase acétate d'éthyle (la phase DMSO/H₂O contient seulement des traces du produit recherché). La phase organique est lavée avec 100 ml d'une solution de NaHCO₃, étape suivie par un lavage avec 100 ml de H₂O ; la phase organique est ensuite séchée avec du MgSO₄ anhydre. Le procédé est répété deux fois avec les deux autres aliquotes restants de 250 ml du mélange réactionnel.

La phase d'acétate d'éthyle est évaporée à l'aide de l'évaporateur rotatif, il reste un liquide visqueux légèrement orange comme du miel (contenant une petite quantité de DMSO). Les résidus de DMSO sont retirés à 100°C sous pression réduite. Une petite quantité d'acétone (10 ml) est ajoutée suivie de 40 ml de diéthyéther. Le solide devient immédiatement blanc crème, il est filtré sur un filtre en céramique. Le thiol résiduel est enlevé du produit réactionnel par chromatographie sur colonne en utilisant hexane/CH₂Cl₂/acétate d'éthyle/méthanol (rapports pondéraux 4/2/1/1) comme phase mobile.

On obtient ainsi 13,1 g (soit un rendement d'environ 89%) du Composé 5.

L'analyse RMN donne les résultats suivants :
*¹H NMR (500 MHz) DMSO-d₆ : 6.93-6.95 (d, 4H), 7.17-719 (d, 4H), 7.42-7.44 (d, 4H), 7.58-7.60 (m, 2H), 7.65-7.68 (m, 1H), 8.49-8.50 (d, 4H), 8.61-8.63 (d, 2H), 10.04 (s, 2H).*

La masse moléculaire du produit telle que mesurée par spectrométrie de masse "MALDI" (*Matrix-assisted Laser Desorption*/*Ionisation*) (mode positif ; matrice dithranol) est égale à 558,1 (valeur théorique calculée égale à 557,7).

### V-3. Synthèse du Polymère 1

Cet exemple décrit de manière détaillée la synthèse du Polymère 1 selon un procédé déjà commenté à la figure 3, à partir du Monomère A1 (ou Composé 3) conforme à l'invention et du Monomère B1 (ou Composé 5) non conforme à l'invention tels que précédemment décrits, ce Polymère 1 étant obtenu ici d'une part sous forme sulfoné et d'autre part bloqué par des groupements benzophénone.

Le monomère A1 et Na₂CO₃ sont tout d'abord séchés à 150°C toute la nuit (sous vide), séparément, puis ils sont mélangés ensemble pendant 1 h à 160°C. Le Monomère B1 est lui aussi séché à 80°C (sous vide) toute la nuit.

La polymérisation est conduite dans un ballon tricol de 100 ml. Le ballon est surmonté d'une entrée d'azote, d'un thermomètre, d'un agitateur et d'un séparateur "Dean Stark" surmonté d'un réfrigérant. Les parties en verre de l'appareillage (incluant le réfrigérant et le "Dean Stark") sont séchés sous vide à l'aide d'un pistolet à air chaud. Le ballon est chargé avec le Monomère B1 (0,848 g soit 1,52 mmol), le Monomère A1 (1,22 g soit 1,52 mmol), du carbonate de sodium anhydre (0,48 g soit 4,57 mmol ; excès de trois fois), du N,N-diméthylacétamide sec (DMA) (20 ml) et toluène (4 ml ; agent azéotropique). Le ballon de réaction est chauffé dans un bain d'huile à 100°C. La température du bain d'huile est ensuite augmentée à environ 148°C et le toluène résiduel est distillé (140°C à l'intérieur du ballon de réaction).

La trappe du "Dean Stark" est vidée (toluène extrait) et la température du bain d'huile est augmentée à environ 159°C (environ 150°C à l'intérieur du ballon), puis maintenue à cette température durant toute la nuit (20 h au total).

Puis, la température de la réaction est abaissée à 100°C à l'intérieur (le ballon est monté au-dessus du bain d'huile) puis 4 mg de 4-fluorobenzophénone dissous dans 5 ml de DMA sont injectés dans la réaction à l'aide d'une seringue. La réaction de blocage est ensuite poursuivie dans un bain d'huile réglé à environ 145°C (température interne) pendant 4 h. On laisse le mélange réactionnel refroidir à température ambiante, puis le polymère est versé dans 300 ml de 2-propanol. Le précipité fibreux est récupéré par filtration et séché au four à 80°C toute la nuit (sous vide). Le carbonate de sodium est retiré du polymère par lavage dans 30 ml d'eau et acidifié par addition goutte à goutte de HCl 10% jusqu'à pH 7. Le polymère final ainsi obtenu est séché à 100°C sous vide.

L'analyse RMN donne les résultats suivants :
*¹H NMR (500 MHz) DMSO-d₆ : 7.08-7.09 (d, 2H), 7.17-719 (d, 4H), 7.39-7.40 (d, 4H), 7.62-7.64 (m, 6H), 7.80-7.82 (d, 2H), 7.79-7.85 (m, 8H), 8.27-8.28 (s, 2H), 8.65-8.66 (d, 4H), ), 8.69-8.70 (d, 2H).*

### V-4. Fabrication de membranes PEM

Dans cet essai, des membranes de Polymère 1 sont préparées selon la technique dite de *"solvent casting"* comme décrit ci-après.

Le polymère (625 mg) préalablement dissous dans 8 ml de N,N-diméthylacétamide est filtré à travers un microfiltre (société "Millipore") en PTFE (polytétrafluoroéthylène) ayant une taille de pores de 0,45 µm environ. Puis la solution de polymère ainsi filtrée est coulée dans un moule constitué de deux plaques de verre superposées, la plaque supérieure comportant un évidement (dimensions 9 cm x 9 cm) de profondeur égale à 1 mm ; elle est ensuite chauffée à 50°C pendant 24 h, puis 2 h à 60°C. Puis les traces de solvant organique sont évacuées de la membrane ainsi formée en plongeant cette dernière dans un bain d'eau distillée pendant environ 12 h.

Après séchage final à 60°C pendant 2 h sous vide, une membrane robuste et transparente, d'épaisseur égale à environ 50 µm, est ainsi obtenue, prête pour caractérisation.

### V-5. Caractérisation des membranes PEM

### V-5-A) Conductivité protonique

Pour l'acidification de la membrane (pour rappel, échange du cation M⁺ par H⁺), le Polymère 1 est initialement plongé dans 200 ml de H₂SO₄ (aq.) 1,9 M pendant 2 h. On utilise de l'acide H₂SO₄ distillé deux fois (Sigma Aldrich) pour éviter les traces de métaux. De l'eau distillée est ajoutée ensuite en plusieurs étapes (durée totale environ 12 h) pour atteindre un pH égal à 7 ; la membrane est ensuite conservée ainsi dans l'eau distillée pendant toute la nuit (environ 12 heures).

La conductivité protonique de la membrane exprimée en S/cm (Siemens par centimètre) est déterminée comme indiqué ci-après.

Des membranes en forme de disques de 2 cm de diamètre (épaisseur 50 µm) sont découpées en utilisant un emporte-pièce. La conductivité protonique de la membrane est déterminée par la mesure de la partie réelle (Ohmique) et de la partie imaginaire (Capacitance) de l'impédance complexe, ceci dans la plage de fréquences se situant entre 100 kHz et 10 Hz (avec amplitude de 100 mV AC). Les mesures sont faites avec un potentiostat à impédance/AC (Zahner, Allemagne). Des graphes de Nyquist sont générés par les mesures d'un empilement successif de une, deux, trois et jusqu'à six membranes (totalement humidifiées) prises en sandwich entre deux électrodes en platine de même forme circulaire que les membranes.

Pour chaque mesure, on reporte la valeur interceptant l'axe réel de graphe de Nyquist, c'est-à-dire une valeur de la composante imaginaire de l'impédance à zéro. En général ces points sont alignés sur une droite affine dont la pente détermine directement la valeur de la résistance de la membrane. Son ordonnée à l'origine détermine la résistance de contact entre les membranes et les électrodes en platine. Ces dernières valeurs et la connaissance de l'épaisseur permettent de calculer de manière connue la résistivité de la membrane ; l'inverse de cette valeur est la conductivité.

Ainsi testées, les membranes issues du Polymère 1 ont montré des valeurs de conductivité protonique remarquables, égales à environ 35 mS/cm à 25°C (humidité 100%), soit environ 50% de la valeur de conductivité (environ 70 mS/cm) mesurée sur la membrane commerciale ("Nafion® 112") optimisée quant à elle de longue date, de même épaisseur et testée rigoureusement dans les mêmes conditions.

### V-5-B) Capacité d'absorption d'eau et stabilité dimensionnelle

Une fois la membrane acidifiée, elle est séchée sous vide à 100°C pendant 12 h. Son poids est immédiatement mesuré avant qu'elle ne capte l'humidité de l'air. Puis les échantillons de membrane sont immergés dans de l'eau distillée à température ambiante jusqu'à saturation (aucune prise de poids supplémentaire due à l'eau n'est alors observée).

La capacité d'absorption de l'eau, exprimée en %, est calculée comme la différence entre les poids de la membrane humide et de la membrane sèche. La stabilité dimensionnelle, également exprimée en %, est le rapport entre la dimension principale de la membrane sèche et la dimension principale de la membrane totalement humidifiée.

On note que les membranes du Polymère 1 ont une capacité d'absorption d'eau égale à environ 20% de leur poids, comparativement à une valeur d'environ 23% pour la membrane commerciale ("Nafion® 112"). Sa stabilité dimensionnelle est égale à 1%, comparativement à une valeur de 7% pour la membrane commerciale témoin.

En d'autres termes, on constate que les membranes issues des monomères conformes à l'invention présentent de manière inattendue non seulement une capacité d'absorption d'eau réduite mais aussi une stabilité dimensionnelle remarquable, autant de facteurs qui sont déterminants pour l'endurance et la stabilité chimique de la membrane en fonctionnement dans une pile à combustible PEM.

### V-5-C) Performance dans une pile à combustible PEM

Les performances des membranes peuvent être testées sur un banc de test pour piles à combustible sur lequel la température, la pression, le débit et l'humidité des gaz peuvent être réglés. Les gaz utilisés sont de l'hydrogène et de l'oxygène purs, à une température de 65°C.

La pile utilisée dans ces essais est constituée d'une seule cellule comportant la membrane de polymère à tester, disposée entre deux couches "GDE" (*Gas-Diffusion Electrode*), deux plaques bipolaires en graphite et deux électrodes standard ("ELE 0107" de Johnson Matthey) ayant une teneur en platine d'environ 0,4 mg/cm².

La membrane à tester est tout d'abord séchée entre deux non-tissés (qualité chambre stérile, "Sontara Micropure 100" - fournisseur DuPont). Elle est ensuite pressée entre deux plaques de verre, à 60°C pendant 3 h. L'assemblage MEA est obtenu par pressage à chaud d'une couche de catalyse de Pt/C disposée de chaque côté de la membrane (115°C, 125 MPa). A ce stade l'assemblage MEA peut être assemblé entre deux plaques bipolaires pour former une cellule de pile à combustible prête à fonctionner lorsque elle est alimentée en hydrogène et oxygène.

Pour les besoins du test, la pile est soumise à des conditions stationnaires (0,7 V) ou à des situations d'arrêt et démarrage ou "OCV" (*Open Circuit Voltage*), afin de manière connue de soumettre la membrane aux conditions de fonctionnement les plus agressives (e.g. peroxydes, radicaux libres, etc.) et d'en déduire sa résistance chimique globale.

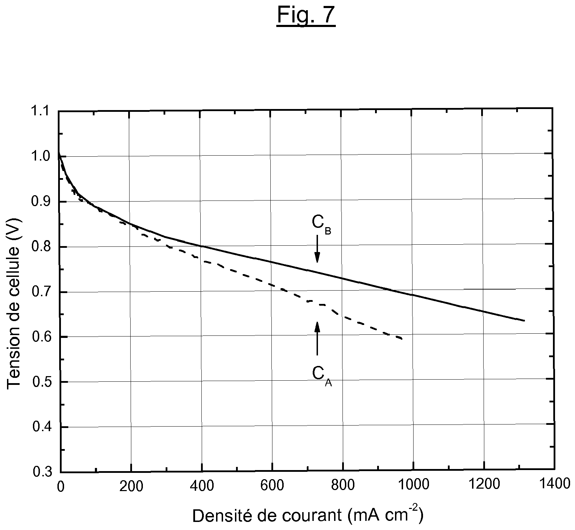

La figure 7 reproduit la courbe dite de polarisation, la tension de cellule étant enregistrée en fonction de la densité de courant délivrée par la pile, d'une part pour la membrane constituée par le Polymère 1 (courbe C_{A}), d'autre part pour la membrane commerciale (polymère "Nafion® 112", courbe C_{B}).

La lecture de ces deux courbes conduit aux commentaires suivants ;
- tout d'abord, à haute tension et courant nul (circuit électrique ouvert), on note que la tension de polarisation est équivalente pour les deux membranes, ce qui illustre pour l'homme du métier une perméabilité aux gaz (O₂ et H₂) équivalente ;
- ensuite, on observe des pentes des deux courbes relativement proches dans leur partie linéaire centrale (typiquement entre 200 et 800 mA/cm²), la pente légèrement inférieure de la courbe C_{A} étant liée à une conductivité ionique moindre de la membrane, ce qui témoigne d'une performance électrique proche des deux membranes, sans même une optimisation particulière des électrodes (anode et cathode) pour la membrane spécifique de l'invention.

En conclusion, les monomères de l'invention permettent de fabriquer des polymères et membranes PEM qui, de manière inattendue, présentent une stabilité chimique et dimensionnelle au moins équivalentes à celles des membranes commerciales du type Nafion® développées pourtant depuis très longtemps, ainsi qu'une conductivité ionique approchant celles de ces membranes commerciales ; ces polymères présentent en outre une stabilité chimique et une résistance à l'oxydation remarquables.

## Revendications

1. Monomère perfluoroalcane aromatique répondant à la formule (I) :
Z₁-Ar₃-C(O)-Ar₁-(CF₂)ₙ-Ar₂-C(O)-Ar₄-Z₂
dans laquelle:
- n est compris dans un domaine de 2 à 8;
- les symboles Ar₁, Ar₂, Ar₃ et Ar₄, identiques ou différents, représentent un groupe phénylène dont l'un au moins est porteur d'au moins un groupe sulfonique -SO₃H ou un groupe sulfonate -SO₃M (M représentant un cation de métal alcalin) ;
- les symboles Z₁ et Z₂, identiques ou différents, représentent un halogène ou un hydroxyle.

2. Monomère selon la revendication 1, l'halogène étant le fluor ou le chlore.

3. Monomère selon la revendication 2, répondant à la formule :
(I-2) F-Ar₃-CO-Ar₁-(CF₂)ₙ-Ar₂-CO-Ar₄-F

4. Monomère selon la revendication 1, répondant à la formule :
(I-3) HO-Ar₃-CO-Ar₁-(CF₂)ₙ-Ar₂-CO-Ar₄-OH

5. Monomère selon l'une quelconque des revendications 1 à 4, dans lequel n est égal à 4.

6. Procédé de synthèse d'un polymère perfluoroalcane par polycondensation d'au moins un monomère perfluoroalcane selon l'une quelconque des revendications précédentes.

7. Utilisation d'un monomère perfluoroalcane selon l'une quelconque des revendications précédentes, pour la fabrication d'une membrane de polymère utilisable dans une pile à combustible du type PEM (Polymer Electrolyte Membrane).

## Patentansprüche

1. Aromatisches Perfluoralkan-Monomer der Formel (I):
Z₁-Ar₃-C(O)-Ar₁-(CF₂)ₙ-Ar₂-C(O)-Ar₄-Z₂
in der:
- n in einem Bereich von 2 bis 8 liegt;
- die Symbole Ar₁, Ar₂, Ar₃ und Ar₄ gleich oder verschieden sind und für eine Phenylengruppe stehen, wovon mindestens eine mindestens eine Sulfonsäuregruppe -SO₃H oder mindestens eine Sulfonatgruppe -SO₃M trägt, stehen, (wobei M für ein Alkalimetallkation steht);
- die Symbole Z₁ und Z₂ gleich oder verschieden sind und für ein Halogen oder ein Hydroxyl stehen.

2. Monomer nach Anspruch 1, wobei das Halogen Fluor oder Chlor ist.

3. Monomer nach Anspruch 2 der Formel:
(I-2) F-Ar₃-CO-Ar₁-(CF₂)ₙ-Ar₂-CO-Ar₄-F

4. Monomer nach Anspruch 1 der Formel:
(I-3) HO-Ar₃-CO-Ar₁-(CF₂)ₙ-Ar₂-CO-Ar₄-OH

5. Monomer nach einem der Ansprüche 1 bis 4, wobei n gleich 4 ist.

6. Verfahren zur Synthese eines Perfluoralkan-Polymers durch Polykondensation mindestens eines Perfluoralkan Monomers nach einem der vorhergehenden Ansprüche.

7. Verwendung eines Perfluoralkan-Monomers nach einem der vorhergehenden Ansprüche zur Herstellung einer Polymermembran, die in einer Brennstoffzelle vom PEM(Polymer Electrolyte membrane)-Typ verwendet werden kann.

## Claims

1. Aromatic perfluoroalkane monomer corresponding to the formula (I):
Z₁-Ar₃-C(O)-Ar₁-(CF₂)ₙ-Ar₂-C(O)-Ar₄-Z₂
in which:
- n is in a range from 2 to 8;
- the symbols Ar₁, Ar₂, Ar₃ and Ar₄ which are identical or different, represent a phenylene group, at least one of them bearing at least one sulphonic group -SO₃H or one sulphonate group -SO₃M, M representing an alkali metal cation;
- the symbols Z₁ and Z₂, which are identical or different, represent a halogen or a hydroxyl.

2. Monomer according to Claim 1, the halogen being fluorine or chlorine.

3. Monomer according to Claim 2, corresponding to the formula:
(I-2) F-Ar₃-CO-Ar₁-(CF₂)ₙ-Ar₂-CO-Ar₄-F

4. Monomer according to Claim 1, corresponding to the formula:
(1-3) HO-Ar₃-CO-Ar₁-(CF₂)ₙ-Ar₂-CO-Ar₄-OH

5. Monomer according to any one of Claims 1 to 4, in which n is equal to 4.

6. Process for the synthesis of a perfluoroalkane polymer by polycondensation of at least one perfluoroalkane monomer according to any one of the preceding claims.

7. Use of a perfluoroalkane monomer according to any one of the preceding claims for the manufacture of a polymer membrane which can be used in a fuel cell of the PEM (Polymer Electrolyte Membrane) type.
